Europäisches Patentamt

European Patent Office

Office européen des brevets

(11) Publication number: 0 366 795
A1

## EUROPEAN PATENT APPLICATION
published in accordance with Art. 158(3) EPC

(21) Application number: 88906062.0

(22) Date of filing: 01.07.88

(86) International application number:
PCT/JP88/00665

(87) International publication number:
WO 89/00287 (12.01.89 89/02)

(51) Int. Cl.5 G01N 27/46 , G01N 27/30

(30) Priority: 03.07.87 JP 165434/87

(43) Date of publication of application:
09.05.90 Bulletin 90/19

(84) Designated Contracting States:
BE DE FR IT

(71) Applicant: Terumo Kabushiki Kaisha
No. 44-1, Hatagaya 2-chome Shibuya-ku
Tokyo 151(JP)

(72) Inventor: YAMAGUCHI, Shuichiro
Terumo Kabushiki Kaisha 1500, Inokuchi
Nakai-cho
Ashigarakami-gun Kanagawa-ken(JP)
Inventor: SHIMOMURA, Takeshi
Terumo Kabushiki Kaisha 1500, Inokuchi
Nakai-cho
Ashigarakami-gun Kanagawa-ken(JP)

(74) Representative: Gillard, Marie-Louise et al
Cabinet Beau de Loménie 55, Rue
d'Amsterdam
F-75008 Paris(FR)

(54) MULTI-SENSOR AND PRODUCTION THEREOF.

(57) This invention relates to a multi-sensor and production thereof equipped, for example, with a plurality of sensors (21), (22), (23), (24) produced by forming a plurality of finely patterned conductive layers (5) on the surface of an insulating substrate (6), covering the surface of the plurality of conductive layers (5) with a plurality of redox function layers (4) exhibiting an oxidation-reduction function and covering further the plurality of redox function layers (4) with a plurality of layers (3) sensitive to different materials. The present invention is applied to clinical analysis and medical studies.

F I G. 1A

S P E C I F I C A T I O N

MULTISENSOR AND METHOD OF MANUFACTURING THE SAME

Technical Field

The present invention relates to a so-called multisensor which is a composite form of a plurality of ion sensors and/or biosensors and a method of manufacturing the same and, more particularly, to a compact multisensor applied to clinical analyses and a method of manufacturing the same.

Background Art

Known examples of conventional individual sensors are ion sensors such as a pH sensor, an $Na^+$ sensor and a $K^+$ sensor and biosensors such as a glucose sensor and a urea sensor, and miniaturization of these sensors has been studied.

In recent years, as miniaturization and multifunctioning of computers have progressed, a demand has arisen for miniaturized, multifunctional and intelligent sensors. For this reason, a so-called multisensor which is a composite form of a plurality of or different types of sensors has attracted attention. Since the multisensor is not merely a group of sensors, problems such as mutual contamination or interference between sensors, a sensor arrangement, isolation, wiring and adhesion of lead wires have been posed. It is therefore difficult to achieve a composite form of sensors.

Examples of a known method of depositing a sensitive layer of a small multisensor are:

(1) lift-off method

(2) inkjet method, and

(3) screen printing

According to the method (1), miniaturization can be easily performed, but manufacture is difficult because complicated lithography steps are required. In the methods of (2) and (3), however, it is difficult to achieve miniaturization.

Disclosure of Invention

It is an object of the present invention to provide a so-called multisensor which is a composite form of a plurality of sensors and a method of manufacturing the same. It is another object of the present invention to provide a compact multisensor which can be used in clinical anaylyses or medical applications.

In order to achieve the above objects, a multisensor of the present invention comprises: a plurality of electrically conductive layers micropatterned on a surface of an isolating substrate; a plurality of redox function layers having a redox function covering surfaces of the plurality of electrically conductive layers; and a plurality of sensitive layers sensitive to concentrations of the same or different types of substances covering the plurality of redox function layers.

A method of manufacturing a multisensor of the present invention comprises the steps of: forming a plurality of micropatterned electrically conductive layers on a surface of an isolating substrate; forming a plurality of redox function layers having a redox function covering surfaces of the plurality of electrically conductive layers; and forming a plurality of sensitive layers sensitive to the same or different types of substances covering the plurality of redox function layers.

Note that the uniformity and formation repetitiveness of the plurality of electrically conductive layers to the isolating substrate surface are called patterning.

In the above arrangement, the plurality of sensitive layers of the multisensor sense concentrations of different substances, information about the sensed concentration is transmitted to the electrically conductive layer via the redox function layer, and the electrically conductive layer generates a voltage response corresponding to the sensed concentration.

In the method of manufacturing a multisensor, a plurality of micropatterned electrically conductive layers are formed on the isolating substrate surface, and redox function layers and sensitive layers sensitive to different substances are coated on the plurality of electrically conductive layers in a one-to-one correspondence.

According to the present invention, there are provided a so-called multisensor which is a composite form of a plurality of sensors and a method of manufacturing the same. In addition, there are provided a compact multisensor which can be used in clinical analyses or medical applications and a method of manufacturing the same.

Brief Description of Drawings

Figs. 1A and 1B are plan and sectional views of a multisensor of Example 1;

Fig. 2A is a plan view showing a pattern of a metal mask;

Fig. 2B is a plan view of an iridium oxide electrode of Example 1;

Fig. 3 is a schematic view showing a measuring apparatus for measuring characteristics of the multisensor;

Fig. 4 is a graph showing electrode characteristics of a glucose sensor of Example 1;

Fig. 5 is a graph showing electrode characteristics of a urea sensor of Example 1;

Fig. 6 is a graph showing electrode characteristics of a penicillin sensor of Example 1;

Fig. 7 is a graph showing electrode characteristics of a pH sensor of Example 1;

Fig. 8 is a schematic view showing an apparatus for conducting a flow injection experiment using the multisensor of Example 1;

Fig. 9 is a timing chart showing results obtained by the experimental example conducted by the apparatus shown in Fig. 8;

Fig. 10 is a plan view showing a multi FET sensor;

Figs. 11A and 11B are plan and sectional views of a multisensor of Example 2;

Fig. 12A is a plan view showing a pattern used in screen printing;

Fig. 12B is a plan view showing a thin carbon film electrode of Example 2;

Fig. 13 is a graph showing characteristics of a pH sensor of Example 2;

Fig. 14 is a graph showing characteristics of an $Na^+$ ion sensor of Example 2;

Fig. 15 is a graph showing characteristics of a $K^+$ ion sensor of Example 2;

Fig. 16 is a graph showing characteristics of a $Cl^-$ ion sensor of Example 2; and

Fig. 17 is a timing chart showing measurement results obtained by Example 4.

Best Mode of Carrying Out the Invention

The present invention will be described in detail below by way of its examples with reference to the accompanying drawings.

<Example 1>

(1)  Formation of Iridium Oxide Electrode

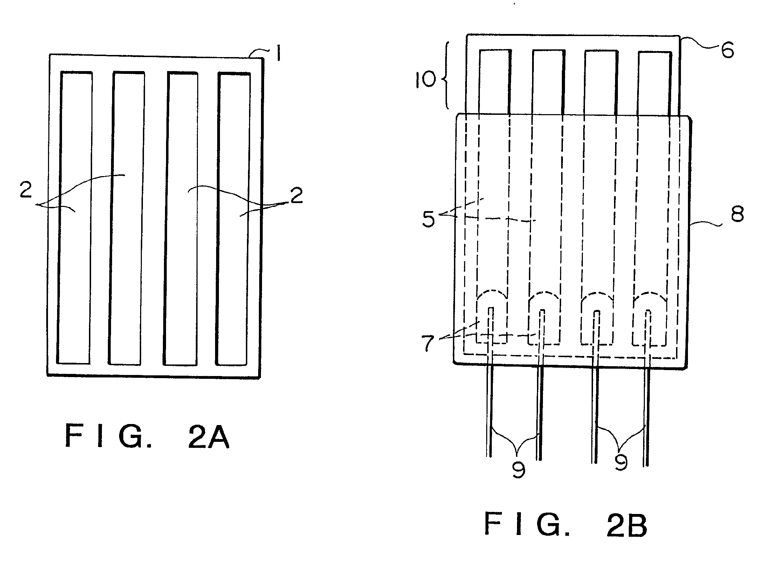

A metal mask 1 (Mo plate) having a pattern shown in Fig. 2A (in which reference numerals 2 denote holes) was urged against a 6 x 10-mm, 0.5-mm thick sapphire substrate 6, and thin iridium oxide film patterns 5 were formed through the metal mask 1 using a sputtering apparatus (SPE-210H: manufactured by NEC Anelva K.K.).

Sputtering conditions:

| | |
|---|---|
| $O_2$ gas | ...0.7 Pa |
| RF power | ...20 W (12.56 MHz) |
| target | ...$IrO_2$ |
| sputtering time | ...60 min |
| film thickness of iridium oxide | ...1,800 to 20,00 $\overset{o}{A}$ |

A lead wire 9 (polyurethane-coated wire having a diameter of 100 mm) was adhered to an end portion of each of the iridium oxide layers 5 formed as described above using an electrically conductive adhesive 7 (C-850-6: manufactured by Amicon Co.).  The iridium oxide layers 5 were electrically isolated using an isolating adhesive 8 (SILICONE (tradename)) except for distal end portions 10 (1 to 2 mm long), thereby forming iridium oxide electrodes as shown in Fig. 2B.

(2)  Formation of Redox Function Layer 4

A redox function layer 4 was coated on the exposed surface of the distal end portion 10 of each iridium oxide layer 5 by an electrolytic polymerization. A 3-electrode cell using the four iridium oxide electrodes the lead wires 9 of which were bound as an active electrode, a saturated sodium chloride calomel electrode (SSCE) as a reference electrode and a platinum steel as a counter electrode, and a potentiostat were used to simultaneously electrolyse the four iridium oxide electrodes.

Electrolyte composition:

| 2,6-dimethylphenol | 0.5 mol/$\ell$ |
| sodium perchloride | 0.2 mol/$\ell$ |
| acetonitrile (solvent) | |

Electrolytic Conditions:

After the electrolytic potential was swept three times from 0 to 1.5 V vs. SSCE in a nitrogen gas atmosphere at a temperature of $-20^{\circ}$C and a sweep rate of 50 mV/sec, constant potential electrolysis was performed at 1.5 V vs. SSCE for ten minutes.

In this manner, the redox function layers 4 (layer thickness: about $10\mu$m) consisting of poly(2,6-dimethylphenol) were formed.

(3) Formation of Sensitive Layer 3

An enzyme layer and an ion-sensitive layer were coated as a sensitive layer 3 on the surface of each redox function layer 4 by an electrolytic polymerization method as will be described below, thereby forming various types of sensors 20 to 23.

(A) Formation of urea sensor 20

In a 3-electrode cell having one of the iridium oxide electrodes as an active electrode, an SSCE as a reference electrode and a platinum steel as a counter electrode, a potentiostat was used in the following electrolyte to sweep the electrolytic potential three times (sweep rate: 50 mV/sec) from 0 to 1.5 V and then constant potential electrolysis was performed at 1.5 V for ten minutes. In this manner, a urease layer (layer thickness: about $50 \mu$m) was coated to form a urea sensor 20.

Electrolyte composition:

| | |
|---|---|
| urease (1 mg/5IU) | 10 mg/m$\ell$ |
| 1,2-diaminobenzene | 20 mol/$\ell$ |
| sodium phosphate | 50 m mol/$\ell$ |
| aqueous solution | (pH 8.04) |

(B) Formation of glucose sensor 21

Following the same procedures as in item (A), one iridium oxide electrode was electrolysed in the following GOD (glucose oxidase) electrolyte to coat a $50-\mu$m thick GOD layer, thereby forming a glucose sensor 21.

Electrolyte composition:

| | |
|---|---|
| glucose oxidase | 1 mg/m$\ell$ |
| 1,2-diaminobenzene | 20 mM |

|                    |            |
| ------------------ | ---------- |
| sodium perchlorate | 0.5 M      |
| aqueous solution   | (pH 6.5)   |

(C)   Formation of penicillin sensor 22

Following the same procedures as in item (A), one iridium oxide electrode was electrolysed in the following electrolyte for a penicillinase layer to coat a 50-$\mu$m thick penicillinase layer, thereby forming a penicillin sensor 22.

Electrolyte composition:

|                     |                      |
| ------------------- | -------------------- |
| penicillinase       | 1,200 I.U./m$\ell$   |
| 1,2-diaminobenzene  | 20 mM                |
| sodium perchlorate  | 0.5 M                |
| aqueous solution    | (pH 7.0)             |

(D)   Formation of pH sensor 23

Following the same procedures as in item (A), one iridium oxide electrode was used to cause an electrolytic polymerization reaction in an electrolyte not containing an enzyme to form a poly(1,2-diaminobenzene) layer, thereby forming a pH sensor 23.

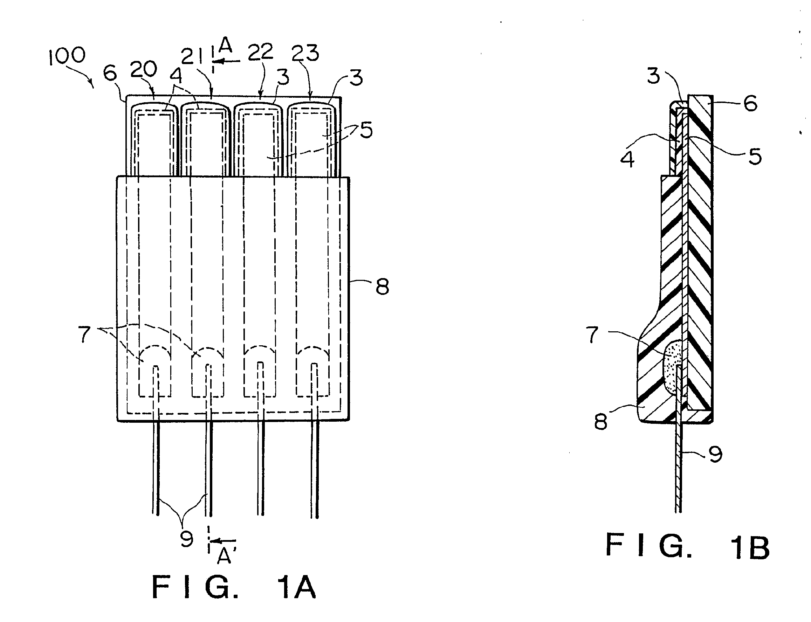

Fig. 1A shows an arrangement of the multisensor according to this example, and Fig. 1B is a sectional view taken along a line A - A' in Fig. 1A.  The sensors 20 to 23 are summarized in Table 1 below.

Table 1

| Sensor-No. | Sensor Type | Sensitive Layer |
|------------|-------------|-----------------|
| 20 | Urea Sensor | (A) Urease Layer |
| 21 | Glucose Sensor | (B) GOD Layer |
| 22 | Penicillin Sensor | (C) Penicillinase Layer |
| 23 | pH Sensor | (D) Hydrogen Ion Carrier Layer |

<Experimental Example 1>

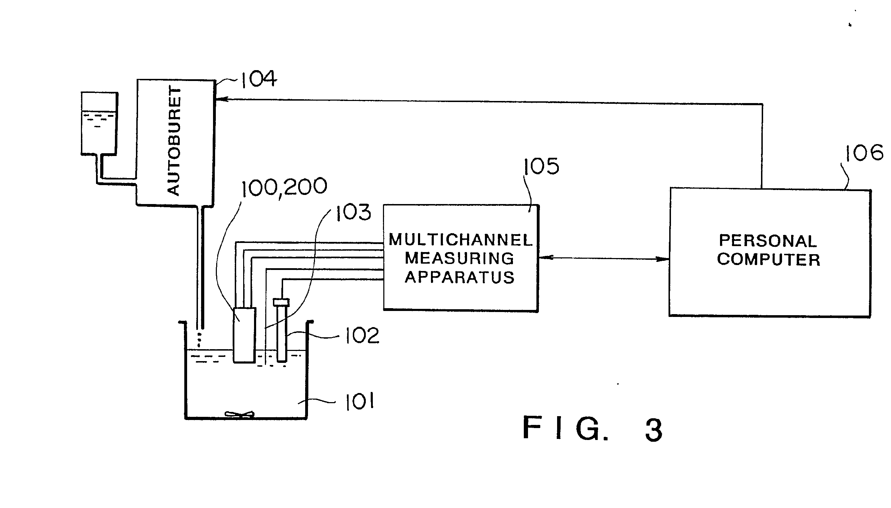

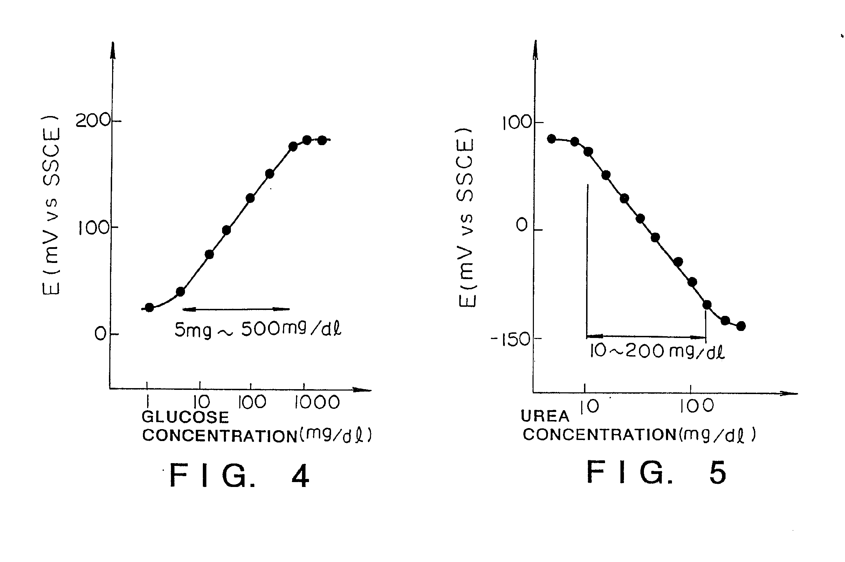

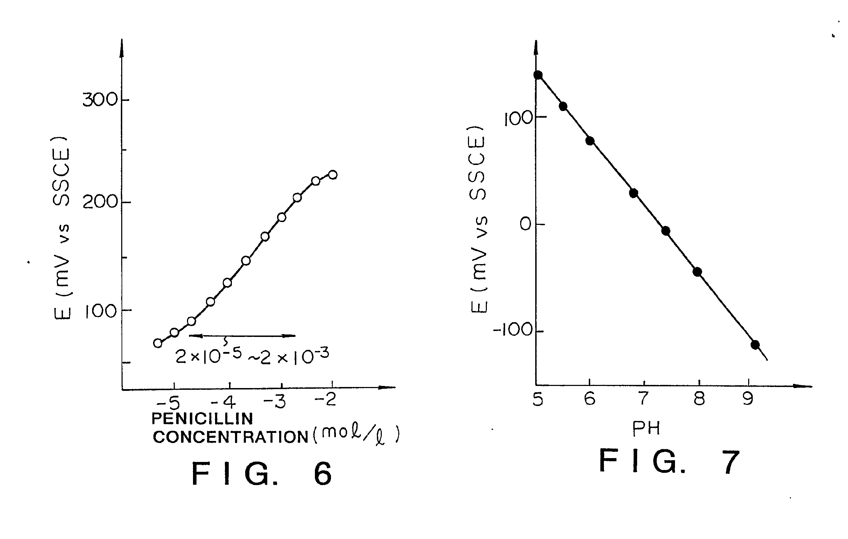

Only a sensitive portion of a multisensor 100 prepared in this example was dipped together with a reference electrode (SSCE) 102 and a common electrode (silver wire) 103 in a sample solution 101 of a measuring apparatus shown in Fig. 3. Using an autoburet 104, a solution containing substrates (glucose, urea and penicillin) of known concentrations was added in the sample solution to change the substrate concentrations. Responses were measured by a multichannel measuring apparatus 105 having a differential electrometer. The measurement values were supplied to and recorded by a personal computer 106 and then analyzed to check the responses to the substrate concentration changes. The check results are shown in Figs. 4 to 7.

As shown in Figs. 4 to 7, the multisensor of this example has good linearity when the glucose, urea and penicillin concentrations and the pH fall within the ranges of 5 to 500 mg/d$\ell$, 10 to 200 mg/d$\ell$, $2 \times 10^{-5}$ to $2 \times 10^{-3}$ mol/$\ell$ and 5.0 to 9.0, respectively.

<Experimental Example 2>

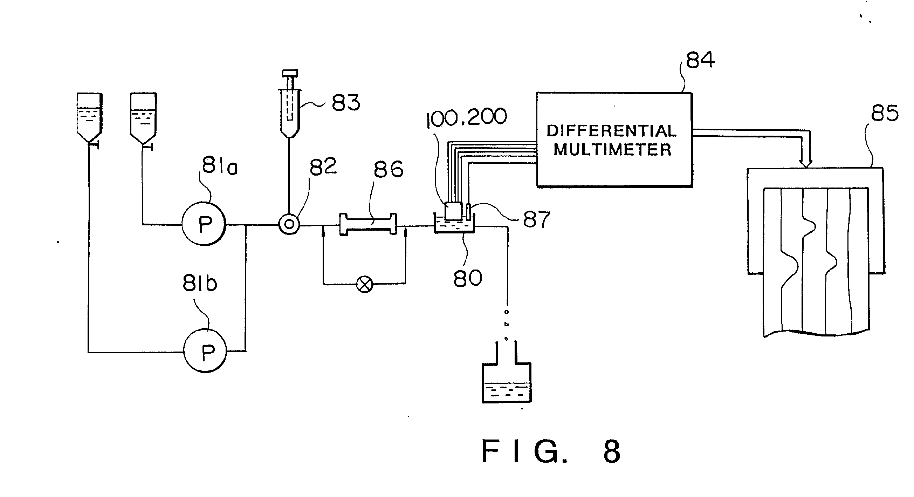

The multisensor 100 of this example was placed in a flow through cell 80 shown in Fig. 8, and a buffer solution (tris-hydrochloric acid-based buffer solution having a pH of 7.4 or 8.0) was flowed at a flow rate of 0.5 mℓ/min using metering pumps 81a and 81b. Outputs from the multisensor were measured by a multichannel measuring apparatus 84 and recorded by a multipen recorder 85.

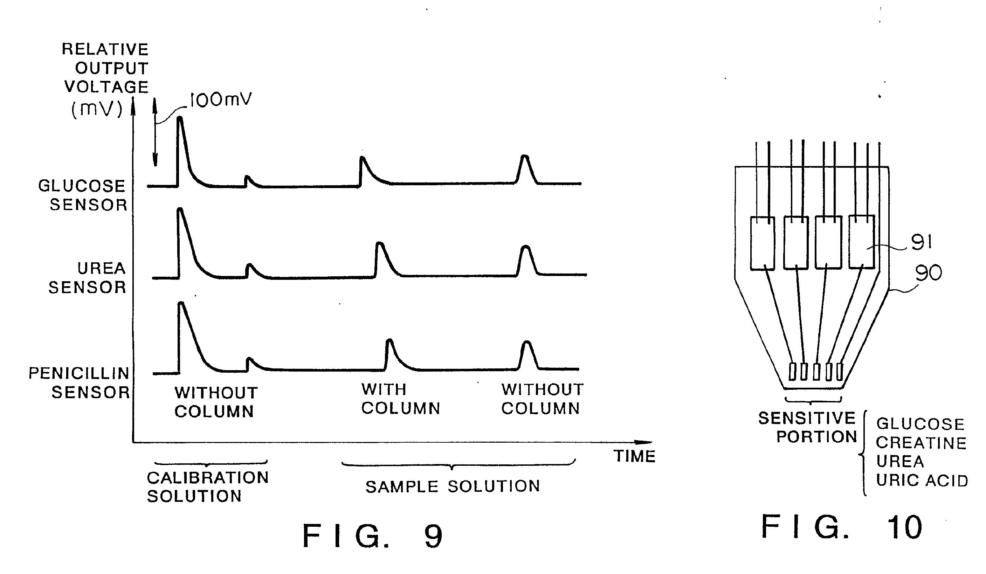

A calibration solution containing three substrates (glucose, urea and penicillin) of known concentrations was injected from an injector 83 via an injector valve 82 to calibrate the multisensor 100, and then a sample solution of an unknown concentration was injected. Outputs from the multisensor 100 together with calibration curves are shown in Fig. 9.

As shown in Fig. 9, regardless of whether or not the solution was separated (eluted) through a column 86, substantially the same outputs could be obtained from the multisensor of this example.

According to the multisensor of this example, since the sensitive layers are formed on the patterned base plate using the electrolytic polymerization method, a multisensor comprising a plurality of sensors can be easily prepared by simple manufacturing steps. In addition, if the multisensor of this example is used as a detector for a flow injection analyzer, a large number of pieces of information can be simultaneously obtained without a column.

The present invention is not limited to the arrangement of the above examples. As shown in Fig. 10, for example, a MOSFET or J-FET may be formed or an FET chip may be bonded on a single isolating substrate to form a multi FET sensor. As a result, a further miniaturized multisensor can be provided.

<Example 2>

(1) Formation of thin carbon layer electrode

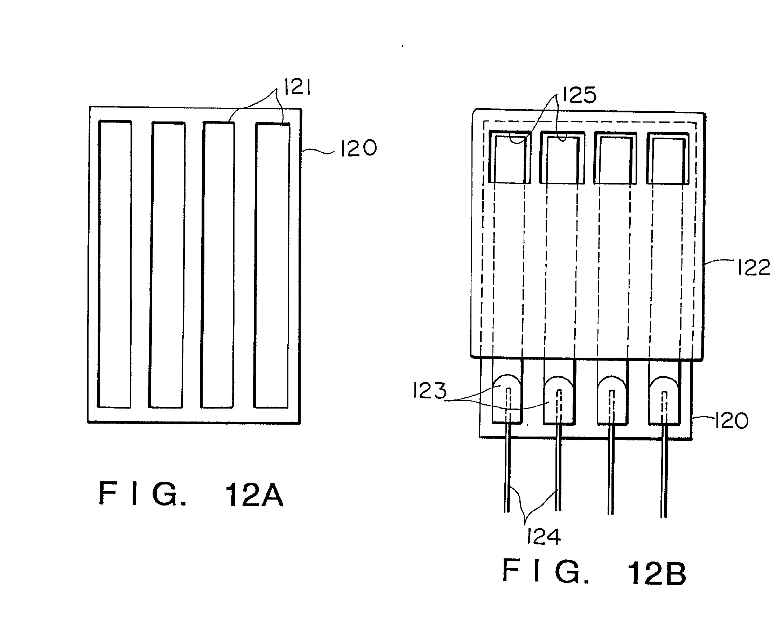

A carbon paste (JEF-010: manufactured by Japan Atison K.K.) was coated using a screen printer and dried at 150°C for 30 minutes to prepare a substrate 120 having thin carbon layers 121 (layer thickness: $2\mu$m) having a pattern shown in Fig. 12A.

Then, as shown in Fig. 12B, an isolating coating agent 122 (silicone-based coating agent) was coated (coating thickness: $500\mu$m) by the screen printer and dried at 180°C for 30 minutes, and lead wires 124 (polyurethane-coated wires each having a diameter of 100 $\mu$m) were adhered using electrically conductive adhesives 123 (C-850-6: manufactured by Amicon Co.).

(2) Formation of redox function layer 126

The four lead wires 124 of the thin carbon layer electrodes prepared as described above were bound and only exposed portions 125 at the distal ends shown in Fig. 12B were dipped in an electrolyte to form an active electrode. Using a 3-electrode cell having this active electrode, a saturated sodium chloride calomel electrode (SSCE) as a reference electrode and a platinum steel as a counter electrode, and a potentiostat, redox function layers 126 (poly(2,6-ditylphenol) layers) were formed on the surfaces of the four carbon exposed portions 125. The composition of the used electrolyte was as follows.

Electrolyte Composition:

| | |
|---|---|
| 2,6-dimelphenol | 0.5 mol/$\ell$ |
| sodium perchlorate | 0.2 mol/$\ell$ |
| acetonitrile (solvent) | |

Electrolytic conditions

After the electrolytic potential was swept three times from 0 to 1.5 V vs. SSCE in a nitrogen gas atmosphere at a temperature of $-20^{o}C$ and a sweep rate of 50 mV/see, constant potential electrolysis was performed at 1.5 V vs. SSCE.

(3) Coating of ion-sensitive layer 127

Using a microsyringe, adequate amounts (0.5 to $10\mu\ell$ ) of coating solutions containing ion carrier substances having compositions shown in Tables 3 and 4 were repeatedly dropped a several times on the exposed portions 125 shown in Fig. 12B, thereby forming ion-sensitive layers 127 sensitive to a pH and $Na^{+}$, $K^{+}$ and $Cl^{-}$ ions, respectively.

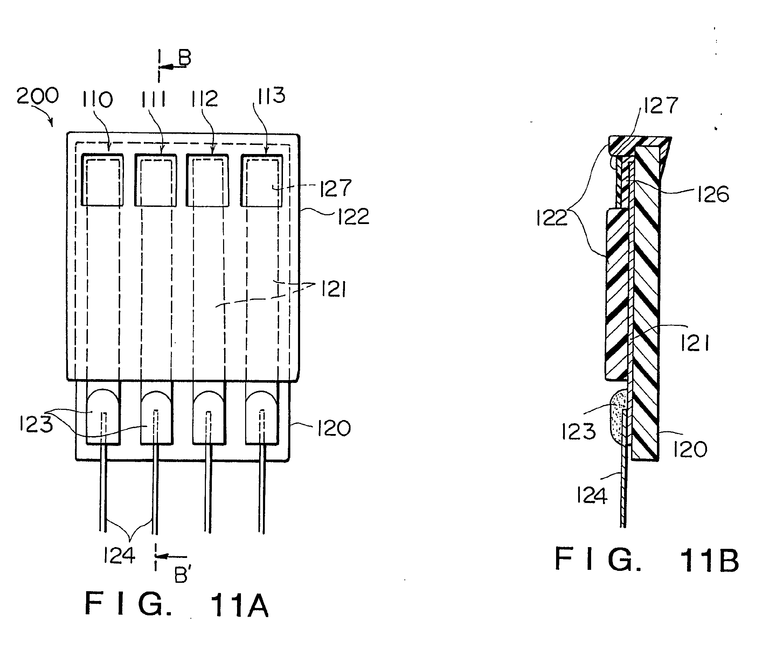

Fig. 11A shows an arrangement of the multisensor of this example, and Fig. 11B is a sectional view taken along a line B - B' in Fig. 11A.

Table 2

| Ion Sensor | Composition | | | Layer |
|---|---|---|---|---|
| 110 | Tridodecylamine | 15.5 | mg/m$\ell$ | pH-sensitive Layer |
| | KTpCl PB | 1.6 | mg/m$\ell$ | |
| | PVC | 81.2 | mg/m$\ell$ | |
| | DOS | 162.8 | mg/m$\ell$ | |
| 111 | Bis(12-crown-4) | 6.25 | mg/m$\ell$ | $Na^+$-sensitive Layer |
| | KTpCl PB | 1.25 | mg/m$\ell$ | |
| | PVC | 80.5 | mg/m$\ell$ | |
| | DOS | 161.0 | mg/m$\ell$ | |

Table 3

| Ion Sensor | Composition | | | Layer |
|---|---|---|---|---|
| 112 | Valinomycin | 6.25 | mg/m$\ell$ | $K^+$-sensitive Layer |
| | KTpCl PB | 1.25 | mg/m$\ell$ | |
| | PVC | 80.5 | mg/m$\ell$ | |
| | DOS | 161.0 | mg/m$\ell$ | |
| 113 | Triphenyl Tin Chloride | 6.28 | mg/m$\ell$ | $Cl^-$-sensitive Layer |
| | PVC | 32.5 | mg/m$\ell$ | |
| | DOS | 72.5 | mg/m$\ell$ | |

Note that THFC (tetrohydrofuran), was used as the solvent.

In the above Tables:

| | |
|---|---|
| KTpCl PB | : potassium tetra(chlorophenyl)borate |
| Bis(12-crown-4) | : bis[(12-crown-4)methyl] methyl |
| PVC | : polyvinyl chloride |
| DOS | : dioctyl sebacate |

<Experimental Example 3>

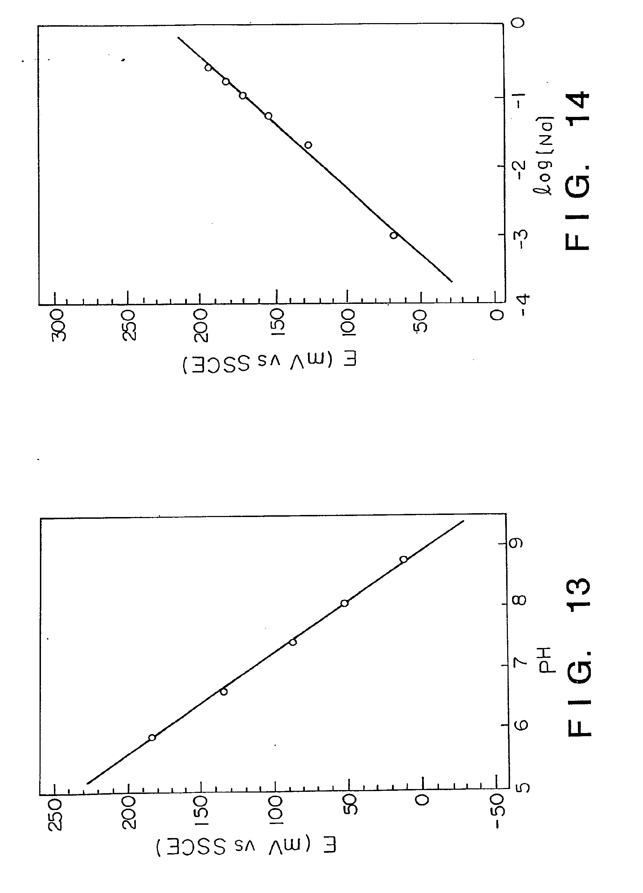

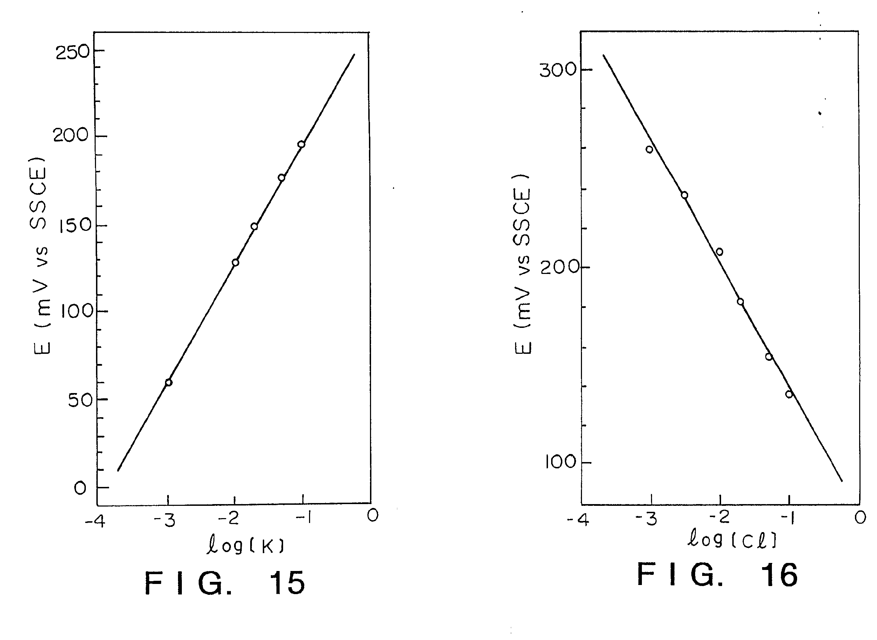

Measurement results of individual sensor characteristics of the multisensor 200 prepared in this example are shown in Figs. 13 to 16. Using the measuring apparatus shown in Fig. 3, in accordance with programed instructions from the personal computer 106, a solution having known concentrations was added from the autoburet 104 in order to change ion concentrations in the sample solution 101, and electromotive forces of the ion sensors with reference to the reference electrode (SSCE) 102 were measured at $37^{o}$C by a multichannel high-input resistance voltmeter. The measurement results were input to the personal computer 106 and then calculated and analyzed. The analyzed results are summarized in Table 4.

<Experimental Example 4>

The result obtained by applying the multisensor of this example to an ion chromatography detector will be described below.

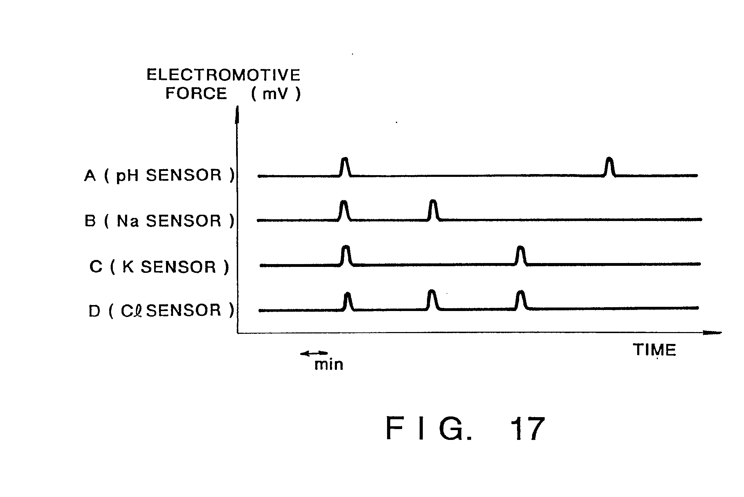

The multisensor 200 and a compact reference electrode 87 were placed in the flow through cell 80 of the apparatus shown in Fig. 8, a buffer solution (pH = 7.4) was flowed at a flow rate of 0.5 m$\ell$/min by the metering pump 81a, and a buffer solution (pH = 6.0), a 0.1-M NaCl solution, a 0.1-M KCl solution and a solution mixture were injected using the injector 83 via the injector valve 82. Obtained electromotive force responses are shown in Fig. 17.

As shown in Fig. 17, the respective ion concentrations in the solution containing a plurality of types of ions can be measured without adversely affecting each other.

Since the multisensor of this example comprises a plurality of micropatterned sensors, it can simultaneously measure a large number of pieces of information regardless of its miniaturized size. In addition, since the multisensor can be prepared utilizing a lithography technique, miniaturization and a composite form of the multisensor can be easily achieved.

Also, a multisensor with a high response speed can be provided. Furthermore, a multisensor without a problem of mutual interference between individual sensors can be provided.

The present invention is not limited to the above examples. As shown in Fig. 10, for example, a MOSFET or J-FET may be formed or an FET chip may be bonded on a single isolating substrate to form a multi FET sensor.

As a result, a further miniaturized multisensor can be provided.

It is to be understood that the present invention includes all the changes and additions that fall within the scope of the appended claims.

C L A I M S

1. A multisensor comprising:

a plurality of electrically conductive layers micropatterned on a surface of an isolating substrate;

a plurality of redox function layers having a redox function covering surfaces of said plurality of electrically conductive layers; and

a plurality of sensitive layers sensitive to the same or different types of substances covering said plurality of redox function layers.

2. A multisensor according to claim 1, wherein said redox function layers are formed by an electrolytic oxidative polymerization method.

3. A multisensor according to claim 1, wherein said sensitive layers are ion-sensitive layers.

4. A multisensor according to claim 1, wherein said sensitive layers are enzyme layers.

5. A multisensor according to claim 3 or 4, wherein said ion-sensitive layers and/or said enzyme layers are formed by an electrolytic polymerization method.

6. A method of manufacturing a multisensor, comprising the steps of:

forming a plurality of micropatterned electrically conductive layers on a surface of an isolating substrate;

forming a plurality of redox function layers having a redox function covering surfaces of said plurality of electrically conductive layers; and

forming a plurality of sensitive layers sensitive to the same or different types of substances covering said plurality of redox function layers.

7. A method according to claim 6, wherein said redox function layers are formed by an electrolytic oxidative polymerization method.

8. A method according to claim 6, wherein said sensitive layers are ion-sensitive layers.

9. A method according to claim 6, wherein said sensitive layers are enzyme layers.

10. A method according to claim 8 or 9, wherein said ion-sensitive layers and/or said enzyme layers are formed by an electrolytic polymerization method.

FIG. 1B

FIG. 1A

F I G. 2A

F I G. 2B

EP 0 366 795 A1

F I G. 3

EP 0 366 795 A1

F I G.  4

F I G.  5

EP 0 366 795 A1

F I G. 6

F I G. 7

EP 0 366 795 A1

FIG. 8

EP 0 366 795 A1

RELATIVE
OUTPUT
VOLTAGE
(mV)

100mV

GLUCOSE
SENSOR

UREA
SENSOR

PENICILLIN
SENSOR

WITHOUT
COLUMN

WITH
COLUMN

WITHOUT
COLUMN

TIME

CALIBRATION
SOLUTION

SAMPLE SOLUTION

F I G. 9

91

90

SENSITIVE
PORTION

GLUCOSE
CREATINE
UREA
URIC ACID

F I G. 10

EP 0 366 795 A1

F I G.  11A

F I G.  11B

EP 0 366 795 A1

F I G. 12A

F I G. 12B

EP 0 366 795 A1

FIG. 14

E (mV vs SSCE)

log(Na)

FIG. 13

E (mV vs SSCE)

PH

FIG. 15

FIG. 16

EP 0 366 795 A1

ELECTROMOTIVE
FORCE  ( mV )

A ( pH SENSOR )

B ( Na SENSOR )

C ( K SENSOR )

D ( Cℓ SENSOR )

min

TIME

F I G.  17

EP 0 366 795 A1

# INTERNATIONAL SEARCH REPORT

International Application No  PCT/JP88/00665

## I. CLASSIFICATION OF SUBJECT MATTER (if several classification symbols apply, indicate all) 6

According to International Patent Classification (IPC) or to both National Classification and IPC

Int.Cl$^4$    G01N27/46, G01N27/30

## II. FIELDS SEARCHED

| Minimum Documentation Searched 7 | |
|---|---|
| Classification System | Classification Symbols |
| IPC | G01N27/46-27/56, G01N27/30 |

Documentation Searched other than Minimum Documentation
to the Extent that such Documents are Included in the Fields Searched 8

| | |
|---|---|
| Jitsuyo Shinan Koho | 1926 - 1988 |
| Kokai Jitsuyo Shinan Koho | 1971 - 1988 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X | JP, A, 60-243555 (Fuji Photo Film Co., Ltd.) 3 December 1985 (03. 12. 85) & EP, A, 161690 | 1, 3, 6, 8 |
| Y | JP, A, 58-10645 (Fuji Photo Film Co., Ltd.) 21 January 1983 (21. 01. 83) & DE, A, 3226045 & GB, A, 2102963 | 1-10 |
| Y | JP, A, 62-11159 (Massachusetts Institute of Technology) 20 January 1987 (20. 01. 87) (Family: none) | 1-10 |
| Y | JP, A, 61-266952 (Terumo Corporation) 26 November 1986 (26. 11. 86) (Family: none) | 1-10 |
| Y | JP, A, 61-251764 (Terumo Corporation) 8 November 1986 (08. 11. 86) (Family: none) | 1-10 |

* Special categories of cited documents: 10

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search | Date of Mailing of this International Search Report |
|---|---|
| September 14, 1988 (14. 09. 88) | September 26, 1988 (26. 09. 88) |
| International Searching Authority | Signature of Authorized Officer |
| Japanese Patent Office | |

Form PCT/ISA/210 (second sheet) (January 1985)